Europäisches Patentamt

European Patent Office

Office européen des brevets

(19)

(11) Veröffentlichungsnummer : **0 065 271**
**B1**

(12) # EUROPÄISCHE PATENTSCHRIFT

(45) Veröffentlichungstag der Patentschrift :
01.10.86

(21) Anmeldenummer : 82104133.2

(22) Anmeldetag : 12.05.82

(51) Int. Cl.⁴ : **G 05 D 11/13// A61M16/00**

(54) **Verfahren zum Mischen von Gasen in einem vorgegebenen Verhältnis und zum Dosieren der Gasmischung.**

(30) Priorität : 14.05.81 SE 8103025

(43) Veröffentlichungstag der Anmeldung :
24.11.82 Patentblatt 82/47

(45) Bekanntmachung des Hinweises auf die Patenterteilung : 01.10.86 Patentblatt 86/40

(84) Benannte Vertragsstaaten :
DE FR GB IT

(56) Entgegenhaltungen :
DE-A- 2 455 751
DE-A- 2 831 856
US-A- 4 062 373
US-A- 4 162 689
PHILIPS APPLICATION NOTE 31: November 1968,
Eindhoven (NL); "Chemical blending plant control
using 50-series modules"
REGELUNGSTECHNISCHE PRAXIS, Band 20, Nr. 1,
Januar 1978, Seiten 20-21, Munchen (DE); H. SCHLEM-
MINGER: "Temperaturkonpensation bei digitalen
Mischungsregelungen"
(73) Patentinhaber : **Siemens Aktiengesellschaft Berlin
und München
Wittelsbacherplatz 2
D-8000 München 2 (DE)**

(72) Erfinder : **Olsson, Sven-Gunnar
Postlada 652
S-240 17 Soedra Sandby (SE)**
Erfinder : **Jonson, Björn
Nicoloviusvaeg 11
S-223 65 Lund (SE)**
Erfinder : **Neuman, Hanna
Raettervaegen 2
S-171 52 Solna (SE)**

EP 0 065 271 B1

## Beschreibung

Die Erfindung betrifft ein Verfahren zum Mischen von Gasen in einem vorgegebenen Verhältnis und zum Dosieren der Gasmischung und ist speziell zum Mischen von Gasen, die bei der Anästhesie oder der Respiratorbehandlung verwendet werden, vorgesehen.

Ein bekanntes Verfahren zum Mischen von Gasen für die Anästhesie oder die Respiratorbehandlung verwendet mechanische Apparate, die Differenzialdruckregulatoren und Drosselanordnungen enthalten. Diese erlauben keine grössere Genauigkeit als ungefähr ± 3 % und funktionieren nur in einem begrenzten Gasflussbereich ; die Funktion ist bei geringem Gasfluss unzuverlässig. Um beim Mischen von kleinen Gasflüssen eine ausreichende Genauigkeit zu erhalten, muss in gewissen Fällen ein grösserer Gasfluss durch einen Mischer geleitet werden, wobei ein grosser Teil der Gasmenge nicht ausgenutzt werden kann. Ausserdem ist eine derartige Apparatur nicht zuverlässig, da die Funktion durch in die Apparatur gelangenden Schmutz o. dgl. gestört werden kann. Durch eine derartige Funktionsstörung können die Gasquellen, die an die Apparatur angeschlossen sind, in direkten Kontakt miteinander kommen, so dass Gas von einer Gasquelle mit höherem Druck in eine Gasquelle mit niedrigem Druck strömen kann, wenn die Gasmischung nicht aus der Apparatur entnommen wird. Das kann katastrophale Folgen mit sich führen, beispielsweise wenn es sich um Lachgas und Sauerstoffgas handelt, die beide geruchlos sind und daher leicht verwechselt werden können. Ausserdem muss die Dosierung mit einer speziellen Apparatur durchgeführt werden, die nicht Bestandteil der Mischungsanordnung ist, beispielsweise mit einem nach der Mischungsanordnung eingesetzten Rotarmeter mit verstellbarem Drosselventil.

Ein anderes bekanntes Verfahren besteht darin, dass die Gase mit Hilfe von individuellen Rotarmetern für jedes Gas mit in Reihe geschalteten verstellbaren Drosselventilen direkt gemischt werden. In der Praxis kann mit einer solchen Anordnung keine bessere Genauigkeit als ± 10 % erreicht werden. Um eine Gasmischung im gewünschten Verhältnis zu bekommen, muss der notwendige Fluss für jedes Gas berechnet und individuell eingestellt werden.

Bei der Anästhesie besteht ein Bedarf darin, einerseits die Konzentration der verschiedenen Gase in einer Gasmischung und andererseits den Fluss dieser Gasmischung registrieren zu können. Weiterhin liegt ein Bedarf an Alarmeinrichtungen vor, die anzeigen, wann die Zusammensetzung der Gasmischung oder das Volumen des Gasflusses nicht mit den eingestellten Werten übereinstimmt. Dieser Bedarf kann mit den Apparaten, die auf den beiden oben angegebenen Verfahren basieren, nicht befriedigt werden, sondern nur durch Einschalten teurer elektronischer Messapparaturen.

Aus der DE-B2-2 831 856 ist zwar bereits ein Verfahren bekannt, bei dem die Gase von verschiedenen Gasquellen über separate Leitungen impulsweise über steuerbare Ventile einer gemeinsamen Leitung zugeführt werden. Hiermit lassen sich bereits auch kleine Gasflüsse mit ausreichender Genauigkeit mischen. Es ist dazu jedoch noch notwendig, den Gasdruck in jeder Leitung vor den Ventilen und teilweise auch nach diesen zu messen oder zu berechnen. Ein Fehler in einem einzigen Druckmesser oder in einem einzigen Ventil führt automatisch zu einer falschen Mischung der Gase, ohne dass das angezeigt wird.

Der vorliegenden Erfindung liegt die Aufgabe zugrunde, ein genaues, sicheres und zuverlässiges Verfahren zum Mischen und Dosieren von Gasen unter gleichzeitiger Eleminierung der eingangs angegebenen Nachteile zu ermöglichen. Zu diesem Zweck enthält das Verfahren gemäss der Erfindung die im Patentanspruch 1 angegebenen Kennzeichen.

Durch Bestimmung der Gasmengen der einzelnen Gasimpulse in einem für alle Gase gemeinsamen Durchflussmesser und die Steuerung der Ventile über die Messwerte dieses Durchflussmessers ist bereits eine einfache und sichere Regelung möglich. Für Flüssigkeiten ist eine vergleichbare Regelung bereits aus der Philips Appl. Note 31, Nov. 1968, Seite 1 und 2 bekannt. Durch die erfindungsgemäss vorgesehene Verwendung eines weiteren Durchflussmessers, der auf ein und dasselbe Gas anders reagiert als der erste, und den Vergleich der Unterschiede zwischen den Messwerten der beiden Durchflussmesser mit einem vorgegebenen Unterschied wird die Sicherheit des Gasmischungsverfahrens weiter verbessert.

Mit dem erfindungsgemässen Verfahren wird damit die Möglichkeit zum Mischen von beliebig vielen Gasen mit beibehaltener Genauigkeit, Sicherheit und Zuverlässigkeit geschaffen. Weiterhin wird durch die Erfindung auf einfache Weise ermöglicht abzufühlen, ob das richtige Gas zugeführt wird. Gleichzeitig schafft es die Möglichkeit, den Mischungs- und Dosierungsverlauf zu registrieren. Das kann für die Anästhesie wichtig sein, um im Nachhinein deutlich zu machen, ob irgendein Irrtum begangen wurde oder ob ein Funktionsfehler eine falsche Proportionierung oder Dosierung der Gase verursacht hat. Weiterhin schafft es die Möglichkeit, Alarm- und Blockierungskreise zum Anzeigen flascher Funktionen anzuordnen und die Apparatur beim Vorliegen eines wesentlichen Funktionsfehlers umzustellen oder abzustellen.

Für eine ausführlichere Erläuterung der Erfindung soll das Verfahren im folgenden näher mit Hinweis auf die beigefügten Zeichnungen beschrieben werden, wobei

Figur 1 ein Schema über eine Anästhesieeinheit zur Ausübung des Verfahrens gemäss der Erfindung und

Figur 2 ein Diagramm, welches die von der Anästhesieeinheit abgegebenen Gasimpulse veranschaulicht, zeigen.

Von einer Anzahl Anschlüsse 10A, 10B, 10C, 10D und 10E gehen Rohrleitungen 11A, 11B, 11C, 11D und 11E aus. An die Anschlüsse können unterschiedliche Druckgasquellen zum Zuführen unterschiedlicher Gase zu den verschiedenen Leitungen angeschlossen sein. Diese Gasquellen können z. B. Luft unter hohem Druck, Luft unter niedrigem Druck, reinen Sauerstoff ($O_2$), Lachgas ($N_{20}$) und Sauerstoff, gesättigt mit Halotan ($C_{2HBrClF3}$) oder Etran enthalten. In jeder Leitung ist ein Rückschlagventil 12A, 12B, 12C, .12D und 12E sowie ein Magnetventil 13A, 13B, 13C, 13D und 13E eingesetzt. Nach den Magnetventilen sind die Leitungen an eine gemeinsame Leitung 14 angeschlossen, die über einen Flussmesser 15 an eine Mischkammer 16 angeschlossen ist, von der eine Leitung 17 zu einer Gummiblase oder einer anderen Anordnung zum Zuführen des gemischten Gases zu einem Patienten weiterführt. Der Flussmesser 15 kann von einem Typ sein (Pneumotachograph), bei dem das Gas durch eine Reihe paralleler Kanäle strömt und der Druckabfall über diesen Kanälen gemessen und in einem Differenzialdruckmesser in ein elektrisches Flussignal umgewandelt wird. Die Mischkammer 16, die entweder eine separate Einheit darstellen kann oder auch Teil eines Lungenbeatmungsgerätes, kann von einem Typ sein, der einen mittels einer Federanordnung belasteten Balg enthält und der näher in der schwedischen Patentschrift 358 296 beschrieben ist.

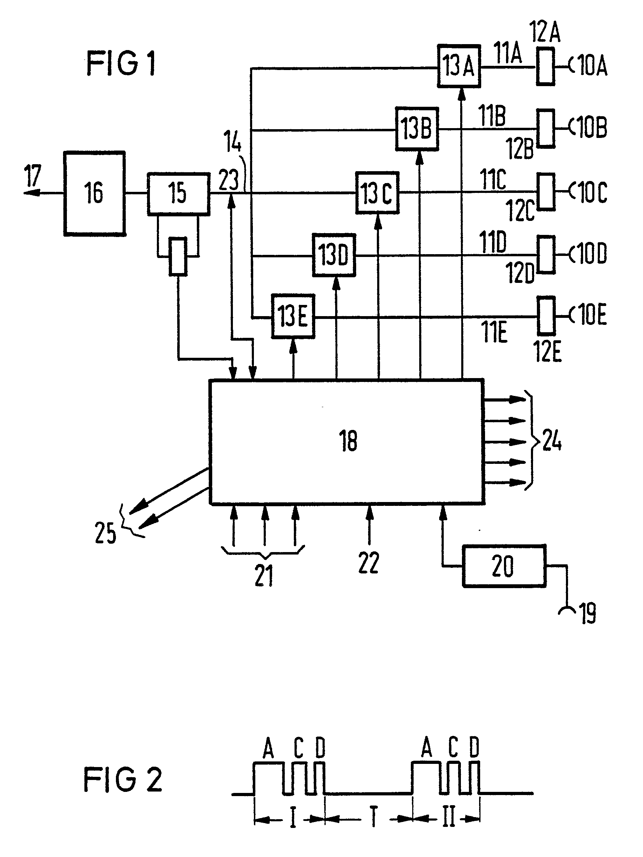

Die Magnetventile 13A bis 13E sind an eine mikroprozessorgesteuerte Regeleinheit 18 angeschlossen, die über das Stromnetz 220/110 Volt, 50/60 Hz, betrieben wird, wobei das Stromnetz bei 19 über ein Akkumulatoraggregat 20 zum Betreiben der Regeleinheit für eine bestimmte Zeit, beispielsweise vier Stunden, wenn der Strom des elektrischen Netzes ausfallen sollte, angeschlossen ist. Die Regeleinheit, die nach bekannten Prinzipien aufgebaut sein kann und dessen Aufbau bei derzeitigen Elektronikkenntnissen nicht im Detail beschrieben zu werden braucht, ist dazu vorgesehen, die Magnetventile 13A bis 13E gemäss einem bestimmten Programm zu öffnen und zu schliessen, um die Gasimpulse von den bei 10A bis 10E angeschlossenen Gasquellen über die Leitungen 11A bis 11E und weiter durch die Leitung 14 über den Flussmesser 15 zur Mischkammer 16 durchzulassen, wo die Gasimpulse gemischt werden für die Weiterbeförderung der Gasmischung durch die Leitung 17 zu einem Patienten. Dabei werden in aufeinander folgenden Impulszügen Gasimpulse abgegeben, wie in Fig. 2 illustriert ist, wo zwei Impulszüge I und II durch das Intervall T voneinander getrennt sind. Jeder hier dargestellte Impulszug besteht aus drei Impulsen, bei denen es sich beispielsweise um einen Impuls A mit Gas von der Quelle 10A, einen Impuls C von der Gasquelle 10C und einen Impuls D von der Gasquelle 10D handeln kann. Die Gasvolumina in den drei Gasimpulsen A, C und D verhalten sich dabei zueinander wie die gewünschten Verhältnisse der Gase von den Gasquellen 10A, 10C und 10D in der gewünschten Gasmischung, die dem Patienten durch die Leitung 17 zugeführt werden soll. Das Mischungsverhältnis der Gasmischung kann dadurch durch Änderung der Gasvolumina in den Impulsen der Impulszüge geändert werden. Die Regeleinheit ist daher mit Organen, schematisch durch die Pfeile 21 markiert, zum Einstellen der Gasvolumina in einzelnen Impulsen und natürlich auch zur Bestimmung davon, welche Gasquellen Gasimpulse zu dem Impulszug abgeben sollen, versehen. Der Gesamtfluss der Gasmischung durch die Leitung 17 wird durch das Einstellen des Intervalles T zwischen den Impulszügen festgelegt. Die Regeleinheit 18 hat ausserdem ein Regelorgan, schematisch durch den Pfeil 22 markiert, zur Einstellung des Flusses auf diese Weise.

Der Durchflussmesser 15 liefert sein Signal an die Regeleinheit 18, wodurch dieses Flussignal als Rückkopplungssignal zum Regeln des Volumens in jedem Gasimpuls dient, wobei jeder Gasimpuls dadurch eingeleitet wird, dass das relevante Regelventil geöffnet wird in Abhängigkeit von dem eingegebenen Programm und abgeschlossen wird, wenn das Signal vom Durchflussmesser 15 anzeigt, dass das vorgesehene Volumen den Durchflussmesser passiert hat.

Jeder einzelne Gasimpuls, unabhängig von welcher Gasquelle er herstammt, wird also durch ein und denselben Durchflussmesser geleitet und in Abhängigkeit von dem Messresultat dieses Durchflussmessers, das der Regeleinheit zugeführt wird, dimensioniert. Ungenauigkeiten oder Fehlerhaftigkeit im Durchflussmesser verändern daher das Mischungsverhältnis in der Gasmischung nicht.

Ein weiterer Vorteil mit einem pulsierenden Gasfluss besteht darin, dass der Durchflussmesser in dem Intervall T zwischen den Impulszügen, wenn also der Gasfluss Null ist, auf Null zurückgestellt werden kann, wodurch der Einfluss einer eventuellen Nullpunktsdrift im Durchflussmesser vermieden werden kann. Nachdem der Durchflussmesser 15 bei jedem Messvorgang von einem Gas bekannter Beschaffenheit und Charakters durchströmt wird, ist es leicht, die Reaktion des Durchflussmessers auf die Unterschiedlichen Eigenschaften der verschiedenen Gase, z. B. zwei so unterschiedliche Viskositäten, im Mikroprozessor der Regeleinheit zu kompensieren. Das Mischungsverhältnis kann bei der Anwendung des Verfahrens gemäss der vorliegenden Erfindung mit grosser Genauigkeit eingestellt werden.

Das Totalvolumen jedes Impulszuges wird also durch den Durchflussmesser 15 mit dem richtigen Verhältnis der betreffenden Gase bestimmt. In Abhängigkeit von diesem Impulszugvolumen wird auch das Intervall T zwischen den Impulszügen bestimmt, so dass sich der vorgesehene Totalfluss der Gasmischung einstellt. Dadurch, dass der Durchflussmesser in den Intervallen auf Null zurückgestellt wird, kann auch der Totalfluss der Gasmischung mit grosser Genauigkeit bestimmt werden.

Die Zufuhr von Impulszügen zur Mischkammer 16 kann in Abhängigkeit von dem in der Mischkammer vorliegenden Volumen und/oder Druck der Gasmischung begonnen und/oder beendet werden, um das Volumen resp. den Druck automatisch in vorgegebenen Grenzen zu halten unabhängig von der Menge der Gasmischung, die der Mischkammer entnommen wird. Die Anschlüsse 10A bis 10E sollten gemäss internationalem Standard gekennzeichnet sein, so dass nur Gasquellen mit Gas der vorgegebenen Beschaffenheit an jeden einzelnen Anschluss angeschlossen werden können. Trotzdem sind schwere Unglücksfälle dadurch vorgekommen, dass Gas von einer Gasquelle zu einer anderen geströmt ist, wie eingangs bereits erwähnt, oder dass eine Gasquelle mit einem falschen Inhalt geliefert wurde. Zur Beseitigung derartiger Fehler ist ausser dem Durchflussmesser 15 ein weiterer Durchflussmesser 23 in der Leitung 14 angeordnet. Dieser Durchflussmesser wird so gewählt, dass er auf die spezifischen Eigenschaften der durchströmenden Gase anders reagiert als der Durchflussmesser 15. Eine passende Ausführungsform eines derartigen Durchflussmessers ist in Acta anaesth. scand. 1979, 23, 349 bis 358 beschrieben. Durch Vergleich der Messresultate der Durchflussmesser 15 und 23 kann bestimmt werden, ob im jeweiligen Fall das richtige Gas die Leitung 14 passiert, da für ein bestimmtes Gas die Messresultate von den beiden Durchflussmessern einen vorbestimmten Unterschied aufweisen. Ein Abweichen von diesem vorbestimmten Unterschied sollte daher ein Blockieren der Anästhesieeinheit veranlassen oder das Auslösen eines Alarmes.

An die Regeleinheit kann ein Display, das durch die Pfeile 24 markiert ist, zur Anzeige des Flusses der Gasmischung sowie der Konzentrationen der unterschiedlichen Gase in der Gasmischung angeschlossen sein. Weiterhin können, wie schematisch durch die Pfeile 25 angedeutet, Anschlüsse für eine Speichereinheit und einer Alarmanordnung zum Registrieren des Mischungs- und Dosierungsverlaufes resp. zur Steuerung der Regeleinheit in Übereinstimmung mit einem gespeicherten Programm und zum Alarmschlagen vorgesehen sein, wenn kritische Abweichungen von den vorgesehenen Werten vorkommen sollten.

## Patentansprüche

1. Verfahren zum Mischen von Gasen in einem vorgegebenen Verhältnis sowie zum Dosieren der Gasmischung, wobei die Gase über separate, mit steuerbaren Ventilanordnungen (13A-13E) versehene Leitungen (11A-11E) einer gemeinsamen Leitung (14) impulsweise nacheinander zugeführt werden und die Impulsgasmengen für die verschiedenen Gase sich zueinander verhalten wie das gewünschte Verhältnis zwischen den Gasen in der Mischung, dadurch gekennzeichnet, dass der Fluss in den einzelnen Gasimpulsen (A, C, D) in zwei Durchflussmessern (15, 23) bestimmt wird, die unterschiedlich auf ein und dasselbe Gas reagieren, dass die Gasmengen der einzelnen Gasimpulse (A, C, D) durch Bestimmung der Längen der Gasimpulse in Abhängigkeit von dem von einem Durchflussmesser (15) erhaltenen Messwert gesteuert werden und dass die Abweichung von einem vorgegebenen Unterschied zwischen den Messwerten der beiden Durchflussmesser (15, 23) operative Gegenmassnahmen anzeigt und/oder auslöst.

2. Verfahren nach Anspruch 1, dadurch gekennzeichnet, dass die Gasimpulse (A, C, D) als aufeinanderfolgende Impulszüge (I, II) zugeführt werden, wobei die Gasmengen für die verschiedenen Gase in jedem Impulszug entsprechend den Verhältnissen zwischen den Gasen in der Mischung dimensioniert sind und wobei das Intervall (T) zwischen den Impulszügen regelbar ist.

3. Verfahren nach Anspruch 2, dadurch gekennzeichnet, dass das Intervall (T) zwischen den Impulszügen (I, II) derart geregelt wird, dass sich ein vorgebbarer mittlerer Fluss der Gasmischung einstellt.

4. Verfahren nach Anspruch 2 oder 3, dadurch gekennzeichnet, dass der zur Messung des Gasflusses verwendete Durchflussmesser (15 resp. 23) in dem Intervall (T) zwischen den Impulszügen (I, II) jeweils auf Null zurückgesetzt wird.

5. Verfahren nach einem der Ansprüche 2-4, dadurch gekennzeichnet, dass die Zufuhr von Impulszügen (I, II) zur gemeinsamen Leitung (14) in Abhängigkeit von dem in dieser Leitung (14) vorliegenden Menge und/oder Druck der Gasmischung bestimmt wird.

## Claims

1. A method of mixing gases in a predetermined proportion and of metering the gas mixture, in which the gases are fed one after another pulsewise to a common pipe (14) through separate pipes (11A-11E) which are provided with controllable valve arrangements (13A-13E), and in which the pulsed gas quantities for the various gases are to one another as the required ratio between the gases in the mixture, characterised in that the flow in the individual gas pulses (A, C, D) is determined in two flowmeters (15, 23) which react differently to one and the same gas ; that the gas quantities of the individual gas pulses (A, C, D) are controlled by determining the lengths of the gas pulses in dependence upon the measured value obtained from one flowmeter (15) ; and that the deviation from a predetermined difference between the measured values of the two flowmeters (15, 23) indicates and/or triggers operative counter-measures.

2. A method as claimed in Claim 1, characterised in that the gas pulses (A, C, D) are supplied as consecutive pulse trains (I, II), the gas quantities for the different gases being metered in each pulse train in accordance with the proportions between the gases in the mixture and the interval (T) between the pulse trains being adjustable.

3. A method as claimed in Claim 2, characterised in that the interval (T) between the pulse trains (I, II) is so adjusted that a predeterminable average flow of the gas mixture is set.

4. A method as claimed in Claim 2 or Claim 3, characterised in that the flowmeter (15 or 23) used for the measurement of the gas flow is in each case reset to zero in the interval (T) between the pulse trains (I, II).

5. A method as claimed in one of Claims 2-4, characterised in that the supply of pulse trains (I, II) to the common pipe (14) is determined in dependence upon the quantity and/or pressure of the gas mixture which is present in said pipe (14).

## Revendications

1. Procédé pour mélanger des gaz en un rapport déterminé à l'avance, ainsi que pour doser le mélange gazeux, les gaz étant amenés les uns après les autres et par impulsion à un conduit commun (14) par des conduits (11A à 11E) distincts, munis de dispositifs à vanne (13A à 13E) pouvant être commandés et les quantités de gaz sous forme d'impulsion pour les divers gaz étant entre elles dans le rapport souhaité entre les gaz dans le mélange, caractérisé en ce qu'il consiste à déterminer le débit dans les impulsions individuelles de gaz (A, C, D) dans deux débitmètres (15, 23) qui réagissent différemment à un seul et même gaz, à commander les quantités de gaz des impulsions individuelles de gaz (A, C, D) en déterminant la longueur des impulsions de gaz en fonction de la valeur de mesure obtenue par un débitmètre (15) et en ce que l'écart, par rapport à une différence donnée à l'avance, entre les valeurs de mesure des deux débitmètres (15, 23) indique et/ou déclenche des contre-mesures opératoires.

2. Procédé suivant la revendication 1, caractérisé en ce qu'il consiste à envoyer les impulsions de gaz (A, C, D) sous la forme de trains d'impulsions (I, II) successifs, les quantités des divers gaz dans chaque train d'impulsions correspondant au rapport entre les gaz dans le mélange et l'intervalle de temps (T) entre les trains d'impulsions pouvant être réglés.

3. Procédé suivant la revendication 2, caractérisé en ce qu'il consiste à régler l'intervalle de temps (T) entre les trains d'impulsions (I, II), de manière à établir un débit moyen du mélange gazeux, qui peut être donné à l'avance.

4. Procédé suivant la revendication 2 ou 3, caractérisé en ce qu'il consiste à remettre à zéro le débitmètre (15, 23) utilisé pour mesurer le débit gazeux dans l'intervalle de temps (T) entre les trains d'impulsions (I, II).

5. Procédé suivant l'une des revendications 2 à 4, caractérisé en ce qu'il consiste à déterminer l'arrivée des trains d'impulsions (I, II) au conduit commun (14) en fonction de la quantité du mélange gazeux se trouvant dans ce conduit (14) et/ou de la pression du mélange gazeux régnant dans ce conduit (14).

FIG 1

FIG 2